# EUROPEAN PATENT APPLICATION

(11) **EP 4 610 998 A1**
(43) Date of publication of application: **03.09.2025**
(21) Application number: 25156592.5
(22) Date of filing: 07.02.2025
(51) Int. Cl.: G16H 40/63, G16H 50/30

(54) **INFORMATION PROCESSING METHOD, APPARATUS AND SYSTEM**

(30) Priority: 27.02.2024 CN 202410218931
(71) Applicant: GE Precision Healthcare LLC, Waukesha, WI 53188 (US)
(72) Inventor: ZHOU, Puzhen, Milwaukee, 53226 (US); HUI, Hui, Milwaukee, 53226 (US)
(74) Representative: Fennell, Gareth Charles

(57) **Abstract**

Provided in embodiments of the present application are an information processing method, apparatus and system. The information processing method is executed by a computer, and comprises: acquiring a value of a vital sign-related parameter of a subject during a preset time period; determining a duration during which the value of the vital sign-related parameter is within a preset value range; and, based on the duration corresponding to the preset value range and a coefficient corresponding to the preset value range, determining vital sign state information of the subject.

## Description

### TECHNICAL FIELD

Embodiments of the present application relate to the technical field of medical devices, and in particular, to an information processing method, apparatus and system.

### BACKGROUND

A monitoring device can monitor vital signs of a subject in real time, and thus may provide important information for medical clinical diagnosis. The monitoring device may set an alert value range, and when a vital sign parameter is within the alert value range, the monitoring device may prompt alert information to a medical worker so that the medical worker learns the current vital sign status of the subject in time.

It should be noted that the above introduction of the background is only for the convenience of clearly and completely describing the technical solutions of the present application, and for the convenience of understanding for those skilled in the art.

### SUMMARY

The inventor has found that a vital sign parameter in an alert value range can only qualitatively indicate that the vital sign parameter is in an abnormal state, but cannot quantitatively reflect a degree of abnormality of the vital sign parameter or a degree of influence on a subject. Therefore, more detailed and accurate information cannot be provided to a medical worker.

To resolve at least one of the above problems, provided in embodiments of the present application are an information processing method, apparatus and system.

According to one aspect of the embodiments of the present application, provided is an information processing method, executed by a computer. The method comprises: acquiring a value of a vital sign-related parameter of a subject during a preset time period; determining a duration during which the value of the vital sign-related parameter is within a preset value range; and, based on the duration corresponding to the preset value range and a coefficient corresponding to the preset value range, determining vital sign state information of the subject.

According to one aspect of the embodiments of the present application, provided is an information processing apparatus, comprising a processor, the processor being configured to execute the information processing method described above.

According to one aspect of the embodiments of the present application, provided is an information processing system. The system comprises: a medical data measurement apparatus, for measuring a value of a vital sign-related parameter of a subject; and the information processing apparatus described above.

One of the beneficial effects of the embodiments of the present application is that: the vital sign state information of the subject is determined based on the duration during which the value of the vital sign-related parameter of the subject is within the preset value range and the coefficient corresponding to the preset value range, and a degree of abnormality of the vital sign-related parameter of the subject during the preset time period can be quantitatively reflected by means of the vital sign state information, so that more detailed and accurate information can be provided for a user.

With reference to the following description and drawings, specific implementations of the embodiments of the present application are disclosed in detail, and the way in which the principles of the embodiments of the present application can be employed are illustrated. It should be understood that the embodiments of the present application are not limited in scope thereby. Within the scope of the spirit and clauses of the appended claims, the embodiments of the present application comprise many changes, modifications, and equivalents.

### BRIEF DESCRIPTION OF THE DRAWINGS

The included drawings are used to provide further understanding of the embodiments of the present application, which constitute a part of the description and are used to illustrate the implementations of the present application and explain the principles of the present application together with textual description. Evidently, the drawings in the following description are merely some embodiments of the present application, and those of ordinary skill in the art may obtain other implementations according to the drawings without involving inventive effort. In the drawings:
FIG. 1 is a schematic diagram of an information processing method according to an embodiment of the present application;
FIG. 2 is a schematic diagram of a trend of a vital sign-related parameter changing with time according to an embodiment of the present application;
FIG. 3 is a schematic diagram of durations according to an embodiment of the present application;
FIG. 4 is a schematic diagram of an information processing apparatus according to an embodiment of the present application; and
FIG. 5 is a schematic diagram of an information processing system according to an embodiment of the present application.

### DETAILED DESCRIPTION

The foregoing and other features of the embodiments of the present application will become apparent from the following description with reference to the drawings. In the description and drawings, specific implementations of the present application are disclosed in detail, and part of the implementations in which the principles of the embodiments of the present application may be employed are indicated. It should be understood that the present application is not limited to the described implementations. On the contrary, the embodiments of the present application include all modifications, variations, and equivalents which fall within the scope of the appended claims.

In the embodiments of the present application, the terms "first", "second", etc., are used to distinguish different elements, but do not represent a spatial arrangement or temporal order, etc., of these elements, and these elements should not be limited by these terms. The term "and/or" includes any and all combinations of one or more associated listed terms. The terms "comprise", "include", "have", etc., refer to the presence of described features, elements, components, or assemblies, but do not exclude the presence or addition of one or more other features, elements, components, or assemblies.

In the embodiments of the present application, the singular forms "a" and "the" include the plural forms, and should be broadly construed as "a type of" or "a class of" rather than being limited to the meaning of "one". Furthermore, the term "the" should be construed as including both the singular and plural forms, unless otherwise specified in the context. In addition, the term "according to" should be construed as "at least in part according to ..." and the term "on the basis of" should be construed as "at least in part based on ...", unless otherwise specified in the context.

In the embodiments of the present application, the term "subject" may be equivalently replaced with "subject under examination", "patient", or "subject of study", which may be a human being, another living being, or the like.

The features described and/or illustrated for one implementation may be used in one or more other implementations in the same or similar way, be combined with features in other embodiments, or replace features in other implementations. The terms "include/comprise" when used herein refer to the presence of features, integrated components, steps, or assemblies, but do not preclude the presence or addition of one or more other features, integrated components, steps, or assemblies.

Description is made below in conjunction with the embodiments.

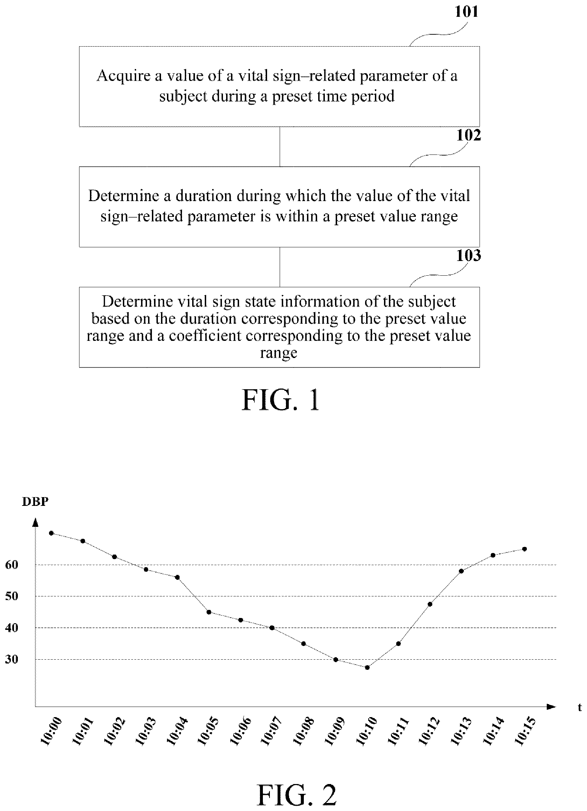

Embodiments of the present application provide an information processing method. The information processing method is executed by a computer. FIG. 1 is a schematic diagram of an information processing method according to an embodiment of the present application. As shown in FIG 1, the method includes:
Step 101: acquiring a value of a vital sign-related parameter of a subject during a preset time period;
Step 102: determining a duration during which the value of the vital sign-related parameter is within a preset value range; and
Step 103: based on the duration corresponding to the preset value range and a coefficient corresponding to the preset value range, determining vital sign state information of the subject.

According to the above embodiment, the vital sign state information of the subject is determined based on the duration during which the value of the vital sign-related parameter of the subject is within the preset value range and the coefficient corresponding to the preset value range, and a degree of abnormality of the vital sign-related parameter of the subject during the preset time period can be quantitatively reflected by means of the vital sign state information, so that more detailed and accurate information can be provided for a user.

In some embodiments, the vital sign-related parameters may include various parameters related to vital signs. For example, the vital sign-related parameters may include: heart rate, blood oxygen saturation, non-invasive blood pressure (systolic, mean and diastolic values), respiratory rate, central venous pressure, invasive arterial pressure (systolic, mean and diastolic values), body temperature, urine volume, brain function, respiratory mechanics, muscle relaxation, blood biochemistry, cardiac output, fractional inspired carbon dioxide pressure (FiCO2), fractional end-tidal carbon dioxide pressure (EtCO2), inspired oxygen concentration (FiO2), end-tidal oxygen concentration (EtO2), and other parameters such as inspired concentration (Fi) value and end-tidal concentration (Et) value of another balance gas or anesthetic gas, entropy (state entropy (SE) and response entropy (RE)), surgical plethysmographic index (SPI), bispectral index (BIS), train-of-four (TOF) ratio, inspiratory peak pressure (Ppeak), plateau pressure (Pplat), positive end-expiratory pressure (PEEP), expiratory minute ventilation (MVexp), expiratory tidal volume (TVexp), anesthetic gas type, anesthetic gas concentration, or anesthetic gas flow rate.

In some embodiments, the preset value range may be a range other than a target value range, wherein the vital sign-related parameter is in a normal state when the value of the vital sign-related parameter is within the target value range; and the vital sign-related parameter is an abnormal state when the vital sign-related parameter is within the preset value range.

Target value ranges for different parameters may be different. For example, a target value range for entropy or a BIS of an anesthetized patient in an operating room is 40 to 60, or a target value range for a mean arterial pressure (from NIBP or IBP) of an ICU patient is 60 mmHg to 100 mmHg, or a target value range for EtCO2 of a mechanically ventilated patient is 30 mmHg to 50 mmHg.

The vital sign-related parameter of the subject being within the target value range means that the subject is in a safe or comfortable state; and the vital sign-related parameter of the subject being within the preset value range means that the subject has a clinical risk, a potential complication, or the like.

In some embodiments, in Step 101, the one or more vital sign-related parameters and values thereof of the subject during the preset time period may be acquired by various means; for example, the parameters may be acquired from a medical data measurement apparatus that measures the value of the vital sign-related parameter of the subject, or may be acquired from a storage apparatus that stores the value of the vital sign-related parameter. This is not specifically limited in the present application.

In some embodiments, the preset time period may correspond to fixed start and end points on a time axis. For example, the preset time period corresponds to 15:10 to 16:15 on a date AAAA-BB-CC.

The present application is not limited thereto, and the preset time period may also correspond to a fixed duration T, a start point and an end point of which on the time axis may be dynamically changed with time. For example, the end point of the preset time period is always the current time, and the start point is a time point T before the current time. For example, if the current time is 16:15 on AAAA-BB-CC and the duration T=15 minutes, the preset time period is 16:00 to 16:15 on AAAA-BB-CC; and every time the current time is increased by one time unit, the start point of the preset time period is correspondingly increased by one time unit. By means of setting the preset time period to a dynamic range, the vital sign state information of the subject can be determined in real time. The time unit may correspond to a sampling interval of the vital sign-related parameter. Sampling intervals of different vital sign-related parameters may be the same or different, and this is not specifically limited in the present application.

In some embodiments, the duration during which the value of the vital sign-related parameter is within the preset value range may be determined in various ways. For example, the duration may include: a cumulative duration within the preset time period during which the value of the vital sign-related parameter is within the preset value range; or, a duration within the preset time period during which the value of the vital sign-related parameter remains continuously within the preset value range in a time dimension.

FIG. 2 is a schematic diagram of a trend of a vital sign-related parameter changing with time according to an embodiment of the present application. In an example in which the vital sign-related parameter is diastolic blood pressure (DBP), a target value range of DBP is 60 mmHg to 90 mmHg. As shown in FIG. 2, a preset time period is 10:00 to 10: 15, wherein from 10:00 to 10:02 and from 10:14 to 10:15, a value of DBP is within the target value range; and from 10:03 to 10:13, the value of DBP is within a preset value range.

The preset value range may include one range. For example, the preset value range may be less than 60 mmHg. In this case, determination results of the above two ways of determining a duration are the same; that is, a cumulative duration during which DBP is within the preset value range is 11 minutes; and a duration during which DBP remains continuously within the preset value range in a time dimension is also 11 minutes.

The preset value range may include a plurality of ranges. Therefore, the accuracy and reliability of the vital sign state information can be improved. For example, the preset value range may include: less than 30 mmHg, 30 mmHg to 40 mmHg (greater than or equal to 30 mmHg and less than 40 mmHg), 40 mmHg to 50 mmHg (greater than or equal to 40 mmHg and less than 50 mmHg), and 50 mmHg to 60 mmHg (greater than or equal to 50 mmHg and less than 60 mmHg).

In an example in which the duration is the cumulative duration during which DBP is within the preset value range: a duration corresponding to the preset value range of less than 30 mmHg is 1 minute (10:10); a duration corresponding to the preset value range of 30 mmHg to 40 mmHg is 3 minutes (10:08, 10:09, and 10: 11); a duration corresponding to the preset value range of 40 mmHg to 50 mmHg is 4 minutes (10:05, 10:06, 10:07, and 10:12); and a duration corresponding to the preset value range of 50 mmHg to 60 mmHg is 3 minutes (10:03, 10:04, and 10:13).

In an example in which the duration is the duration during which DBP remains continuously within the preset value range in the time dimension: a duration corresponding to the preset value range of less than 30 mmHg is 1 minute (10:10); durations corresponding to the preset value range of 30 mmHg to 40 mmHg are 2 minutes (10:08 and 10:09) and 1 minute (10:11); durations corresponding to the preset value range of 40 mmHg to 50 mmHg are 3 minutes (10:05, 10:06, and 10:07) and 1 minute (10:12); and durations corresponding to the preset value range of 50 mmHg to 60 mmHg are 2 minutes (10:03 and 10:04) and 1 minute (10:13).

In some embodiments, at least one of the preset time period, the vital sign-related parameter, and the preset value range may be determined based on input information of a user. Therefore, the user may flexibly select a preset time period, a vital sign-related parameter and a preset value range that interest the user, which can better meet user requirements.

The present application is not limited thereto, and at least one of the preset time period, the vital sign-related parameter and the preset value range may alternatively be determined based on identity information of the subject. Therefore, the preset time period, the vital sign-related parameter, and the preset value range can be automatically set without depending on experience of the user, and user experience can be improved.

In some embodiments, the identity information of the subject may include information such as gender, age, or region of the subject. The present application is not limited thereto, and the identity information of the subject may also include other information related to the vital sign-related parameter.

In some embodiments, the coefficient corresponding to the preset value range of the vital sign-related parameter may be determined based on the input information of the user. Therefore, the user may flexibly set the coefficient corresponding to the preset value range, which can better meet user requirements.

The present application is not limited thereto, and the coefficient corresponding to the preset value range may be automatically generated. For example, in some embodiments, the coefficient corresponding to the preset value range may be related to a distance between the preset value range and a target value range.

For example, a larger distance between the preset value range and the target value range indicates a larger coefficient corresponding to the preset value range; or a smaller distance between the preset value range and the target value range indicates a smaller coefficient corresponding to the preset value range. Therefore, a health risk level of the subject can be represented by a size of the vital sign state information. For example, larger vital sign state information indicates a higher risk level of the subject; and smaller vital sign state information indicates a lower risk level of the subject.

For example, as shown in FIG. 2, the target value range of DBP is 60 mmHg to 90 mmHg, and the coefficient corresponding to the preset value range of less than 30 mmHg may be greater than the coefficient corresponding to the preset value range of 40 mmHg to 50 mmHg. For example, coefficients corresponding to less than 30 mmHg, 30 mmHg to 40 mmHg, 40 mmHg to 50 mmHg, and 50 mmHg to 60 mmHg may be 6, 5, 3, and 1, respectively. The present application is not limited thereto, and the coefficient corresponding to the preset value range may be set based on the distance between the preset value range and the target value range in other ways.

In some embodiments, the coefficient corresponding to the preset value range may also be related to a change trend of the value of the vital sign-related parameter relative to the target value range during a time period during which the value of the vital sign-related parameter is within the preset value range. When the coefficient corresponding to the preset value range is set, the change trend of the value of the vital sign-related parameter is taken into consideration, so that the accuracy and reliability of the vital sign state information of the vital sign-related parameter can be further improved.

In some embodiments, the change trend of the value of the vital sign-related parameter relative to the target value range may include a first trend and a second trend. The first trend may include a trend that the value of the vital sign-related parameter approaches the target value range, and the second trend may include a trend that the value of the vital sign-related parameter deviates from the target value range. A coefficient corresponding to the first trend may be less than or equal to a coefficient corresponding to the second trend.

For example, on the basis of the coefficient corresponding to the preset value range, the coefficient may be modified based on the change trend of the value of the vital sign-related parameter relative to the target value range during the time period during which the value of the vital sign-related parameter is within the preset value range.

For example, the coefficient corresponding to the preset value range of 30 mmHg to 40 mmHg of DBP is 5. If DBP has a trend of gradually increasing during a time period during which DBP is within 30 mmHg to 40 mmHg, the coefficient corresponding to the preset value range of 30 mmHg to 40 mmHg may be modified from 5 to 4.5; or if DBP has a trend of gradually decreasing during the time period during which DBP is within 30 mmHg to 40 mmHg, the coefficient corresponding to the preset value range of 30 mmHg to 40 mmHg may be modified from 5 to 5.5. The present application is not limited thereto, and the coefficient may alternatively be modified in other ways.

In some embodiments, the coefficient corresponding to the preset value range may be further related to a distance between a time when the value of the vital sign-related parameter is within the preset value range and a current time. When the coefficient corresponding to the preset value range is set, the distance between the time when the value of the vital sign-related parameter is within the preset value range and the current time is taken into consideration, so that the accuracy and reliability of the vital sign state information of the vital sign-related parameter can be further improved.

In some embodiments, the value of the vital sign-related parameter is within the preset value range during a first time period and a second time period spaced from the first time period, the first time period being a time period earlier than the second time period. In this case, a coefficient corresponding to the first time period may be less than or equal to a coefficient corresponding to the second time period.

For example, on the basis of the coefficient corresponding to the preset value range, the coefficient may be modified based on the distance between the time when the value of the vital sign-related parameter is within the preset value range and the current time.

For example, the coefficient corresponding to the preset value range of 30 mmHg to 40 mmHg of DBP is 5. During the preset time period, DBP is within 30 mmHg to 40 mmHg during two time periods, for example, DBP is within 30 mmHg to 40 mmHg during two time periods of 10:08 to 10:09 and 10:11. For the time period of 10:08 to 10:09, the coefficient corresponding to the preset value range of 30 mmHg to 40 mmHg may remain unchanged, that is, 5; and for the time period of 10: 11, the coefficient corresponding to the preset value range of 30 mmHg to 40 mmHg may be modified from 5 to 5.1. The present application is not limited thereto, and the coefficient may alternatively be modified in other ways.

In some embodiments, the coefficient corresponding to the preset value range may also be related to a degree of dispersion of the value of the vital sign-related parameter during a time period during which the value of the vital sign-related parameter is within the preset value range. When the coefficient corresponding to the preset value range is set, the degree of dispersion of the value of the vital sign-related parameter during the time period during which the value of the vital sign-related parameter is within the preset value range is taken into consideration, so that the accuracy and reliability of the vital sign state information of the vital sign-related parameter can be further improved.

In some embodiments, a larger degree of dispersion of the value of the vital sign-related parameter during the time period during which the value of the vital sign-related parameter is within the preset value range indicates a larger coefficient corresponding to the preset value range; or a smaller degree of dispersion of the value of the vital sign-related parameter during the time period during which the value of the vital sign-related parameter is within the preset value range indicates a smaller coefficient corresponding to the preset value range.

For example, on the basis of the coefficient corresponding to the preset value range, the coefficient may be modified based on the degree of dispersion of the value of the vital sign-related parameter during the time period during which the value of the vital sign-related parameter is within the preset value range.

For example, the coefficient corresponding to the preset value range of 30 mmHg to 40 mmHg of DBP is 5. During a time period during which DBP is within 30 mmHg to 40 mmHg, if a variance or mean square error of a value of DBP is greater than a preset value, the coefficient corresponding to the preset value range of 30 mmHg to 40 mmHg may be modified from 5 to 5.5; or if the variance or mean square error of the value of DBP is less than or equal to the preset value, the coefficient corresponding to the preset value range of 30 mmHg to 40 mmHg may be modified from 5 to 4.5. The present application is not limited thereto, and the coefficient may alternatively be modified in other ways.

In some embodiments, the coefficient corresponding to the preset value range may also be related to a degree of dispersion of the value of the vital sign-related parameter during the preset time period. When the coefficient corresponding to the preset value range is set, the degree of dispersion of the value of the vital sign-related parameter during the entire preset time period is taken into consideration, so that the accuracy and reliability of the vital sign state information of the vital sign-related parameter can be further improved.

In some embodiments, a larger degree of dispersion of the value of the vital sign-related parameter during the preset time period indicates a larger coefficient corresponding to the preset value range; and a smaller degree of dispersion of the value of the vital sign-related parameter during the preset time period indicates a smaller coefficient corresponding to the preset value range.

For example, on the basis of the coefficient corresponding to the preset value range, the coefficient may be modified based on the degree of dispersion of the value of the vital sign-related parameter during the preset time period.

For example, preset value ranges of DBP are set as follows: coefficients corresponding to less than 30 mmHg, 30 mmHg to 40 mmHg, 40 mmHg to 50 mmHg, and 50 mmHg to 60 mmHg are 6, 5, 3, and 1, respectively. If the variance or mean square error of the value of DBP is greater than a set value during the preset time period, the preset value ranges of DBP may be set as follows: the coefficients corresponding to less than 30 mmHg, 30 mmHg to 40 mmHg, 40 mmHg to 50 mmHg, and 50 mmHg to 60 mmHg are modified from 6, 5, 3, and 1 to 6.4, 5.3, 3.2, and 1.1. If the variance or mean square error of the value of DBP is less than or equal to the preset value, the preset value ranges of DBP may be set as follows: the coefficients corresponding to less than 30 mmHg, 30 mmHg to 40 mmHg, 40 mmHg to 50 mmHg, and 50 mmHg to 60 mmHg are modified from 6, 5, 3, and 1 to 5.6, 4.5, 2.4, and 0.3. The present application is not limited thereto, and the coefficient may alternatively be modified in other ways.

In the above description, the coefficient corresponding to the preset value range may be modified based on the change trend of the value of the vital sign-related parameter relative to the target value range during the time period during which the value of the vital sign-related parameter is within the preset value, or the distance between the time when the value of the vital sign-related parameter is within the preset value range and the current time, or the degree of dispersion of the value of the vital sign-related parameter during the time period during which the value of the vital sign-related parameter is within the preset value range, or the degree of dispersion of the value of the vital sign-related parameter during the preset time period. The present application is not limited thereto, and the coefficient corresponding to the preset value range may alternatively be modified based on a plurality of the change trend of the value of the vital sign-related parameter relative to the target value range during the time period during which the value of the vital sign-related parameter is within the preset value, the distance between the time when the value of the vital sign-related parameter is within the preset value range and the current time, the degree of dispersion of the value of the vital sign-related parameter during the time period during which the value of the vital sign-related parameter is within the preset value range, and the degree of dispersion of the value of the vital sign-related parameter during the preset time period; or, the coefficient corresponding to the preset value range may be modified based on other information.

The foregoing uses an example in which a larger vital sign state information indicates a larger risk level of the subject to provide an exemplary explanation of the coefficient setting. The present application is not limited thereto, and the coefficient may be set such that a larger vital sign state information indicates a lower risk level of the subject.

For example, a larger distance between the preset value range and the target value range indicates a smaller coefficient corresponding to the preset value range; or a smaller distance between the preset value range and the target value range indicates a larger coefficient corresponding to the preset value range; and so on and so forth.

For another example, when the coefficient corresponding to the preset value range may alternatively be modified based on at least one of the change trend of the value of the vital sign-related parameter relative to the target value range during the time period during which the value of the vital sign-related parameter is within the preset value, the distance between the time when the value of the vital sign-related parameter is within the preset value range and the current time, the degree of dispersion of the value of the vital sign-related parameter during the time period during which the value of the vital sign-related parameter is within the preset value range, and the degree of dispersion of the value of the vital sign-related parameter during the preset time period, the coefficient may be modified in a way opposite to the described modification.

For example, the coefficient corresponding to the first trend is greater than the coefficient corresponding to the second trend; or, the coefficient corresponding to the first time period is greater than the coefficient corresponding to the second time period; or, a larger degree of dispersion of the value of the vital sign-related parameter during the time period during which the value of the vital sign-related parameter is within the preset value range indicates a smaller coefficient corresponding to the preset value range, or a smaller degree of dispersion of the value of the vital sign-related parameter during the time period during which the value of the vital sign-related parameter is within the preset value range indicates a larger coefficient corresponding to the preset value range; or, a larger degree of dispersion of the value of the vital sign-related parameter during the preset time period indicates a smaller coefficient corresponding to the preset value range, or a smaller degree of dispersion of the value of the vital sign-related parameter during the preset time period indicates a larger coefficient corresponding to the preset value range.

In some embodiments, in Step 103, the vital sign state information may include a result obtained by weighting the duration corresponding to the preset value range using the coefficient corresponding to the preset value range.

For example, when there is one preset value range, the vital sign state information includes by means of a product of the coefficient corresponding to the preset value range and the duration corresponding to the preset value range.

For another example, when there are a plurality of preset value ranges, the vital sign state information includes a value obtained by performing weighted summation or weighted averaging on the duration corresponding to the preset value range, using the coefficient corresponding to the preset value range. For example: durations corresponding to less than 30 mmHg, 30 mmHg to 40 mmHg, 40 mmHg to 50 mmHg, and 50 mmHg to 60 mmHg are 1 minute, 3 minutes, 4 minutes, and 3 minutes, respectively; if the coefficients corresponding to less than 30 mmHg, 30 mmHg to 40 mmHg, 40 mmHg to 50 mmHg, and 50 mmHg to 60 mmHg are 6, 5, 3, and 1, respectively, the vital sign state information=(1*6)+(3*5)+(4*3)+(3*1)=36.

In some embodiments, one or more vital sign-related parameters may be used to calculate the vital sign state information. For example, when the vital sign-related parameter includes a plurality of parameters, vital sign state information corresponding to each parameter may be determined separately. For example, the vital sign-related parameter includes heart rate and DBP, and vital sign state information of the heart rate and vital sign state information of DBP may be determined, respectively.

Alternatively, when the vital sign-related parameter includes a plurality of parameters, final vital sign state information may be determined based on the vital sign state information corresponding to each parameter and a weight corresponding to each parameter.

In some embodiments, the final vital sign state information includes a value obtained by performing weighted summation or weighted averaging on the vital sign state information corresponding to the parameters by means of the weights corresponding to the parameters.

For example, the vital sign-related parameter includes heart rate and DBP, first vital sign state information determined based on the heart rate is 30, and second vital sign state information determined based on DBP is 42. Assuming that the weight of the heart rate is 3 and the weight of DBP is 2, the final vital sign state information=30*3+42*2=174.

In some embodiments, a weight corresponding to a parameter may be set in various ways. For example, the weight corresponding to the parameter may be related to a degree of influence of the parameter on the subject. For example, a weight of a life safety-related parameter of the subject may be greater than that of a comfort-related parameter of the subject. The present application is not limited thereto, and a weight corresponding to a parameter may alternatively be related to an operation for the subject. For example, the same parameter may have different weights in an anesthesia surgery operation and a nursing care operation.

In some embodiments, the information processing method may further include: when a vital sign-related parameter is selected, information related to the vital sign-related parameter may be displayed or prompted. The related information may include information such as clinical significance information of the vital sign-related parameter to an organ or a vital sign of the subject and/or information about another parameter associated with the vital sign-related parameter, for reference by the user.

For example, heart rate is selected as a vital sign-related parameter, and a system may indicate that heart rate mainly represents a function of a circulatory system, and is also affected by conditions such as anesthetic depth and allergy. The system may also prompt related parameters such as systolic blood pressure, diastolic blood pressure, bispectral index, and entropy index for reference, so that the user can decide whether to add a corresponding vital sign-related parameter.

In some embodiments, the information processing method may further include: displaying or prompting the vital sign state information. For example, a value of the vital sign state information is displayed in real time; and for another example, when the value of the vital sign state information exceeds a preset threshold or the value of the vital sign state information has a trend to continuously increase or decrease, the value of the vital sign state information may be prompted to the user. Therefore, the user can learn the current vital sign state information in time.

In some embodiments, when the vital sign-related parameter includes a plurality of parameters, related parameters may be displayed or prompted together. For example, vital sign state information of parameters having a relatively high correlation is displayed in one area. Therefore, it is easy for the user to view and learn information about the related parameters.

In some embodiments, a duration during which the vital sign-related parameter is within each preset value range, or a percentage of the duration during which the vital sign-related parameter is within each preset value range, may also be displayed. Therefore, more information about the vital sign-related parameter can be provided to the user. For example, the duration or the percentage of the duration during which the vital sign-related parameter is within each preset value range may be displayed in the form of a histogram.

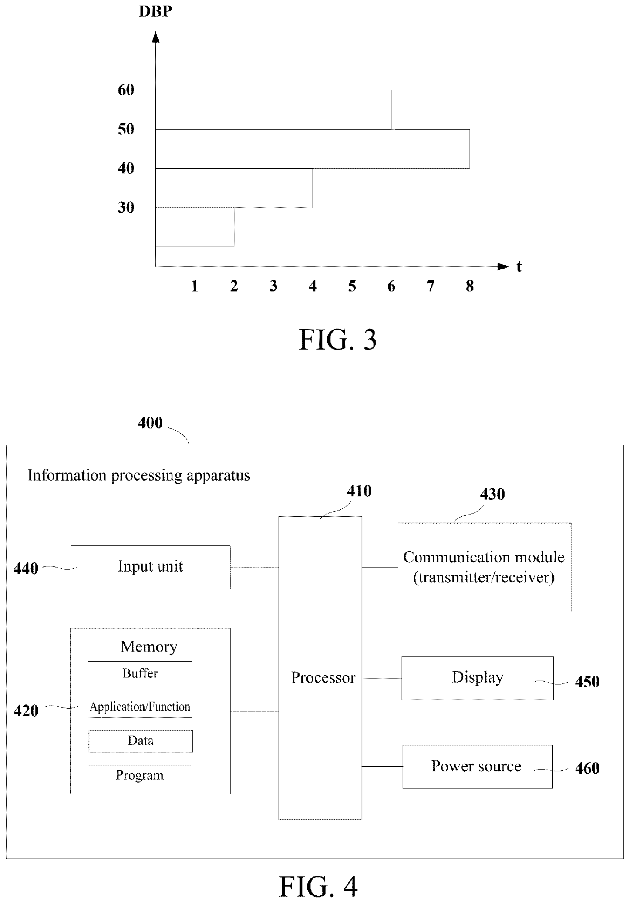

FIG. 3 is a schematic diagram of durations according to an embodiment of the present application. As shown in FIG. 3, during a preset time period, durations during which DBP is within value ranges of less than 30 mmHg, 30 mmHg to 40 mmHg, 40 mmHg to 50 mmHg, and 50 mmHg to 60 mmHg are 2 minutes, 4 minutes, 8 minutes, and 6 minutes, respectively.

In some embodiments, the information processing method may further include: based on the vital sign state information, prompting or adjusting medication information for the subject, a way of monitoring the vital sign-related parameter, or a vital sign-related parameter to be monitored. Thus, an operation suggestion corresponding to the current vital sign state information can be provided to the user, or the user can be assisted to adjust the operation appropriately, thereby further improving the user experience.

For example, medication information, such as medication dosage, medication type, or medication time, corresponding to the current vital sign state information may be prompted to the user, thereby facilitating the user to adjust subject medication in time; or, the way of monitoring the vital sign-related parameter corresponding to the current vital sign state information or the vital sign-related parameter to be monitored may be adjusted, for example, a monitoring period may be prolonged or shortened, and currently monitored vital sign-related parameters may be increased or decreased, so that the information about the vital sign-related parameter may be more appropriately provided to the user.

It should be noted that the above figures merely schematically illustrate the embodiments of the present application, but the present application is not limited thereto. For example, the order of execution between operations may be appropriately adjusted. In addition, some other operations may be added, or some operations may be omitted (for example, operations or steps corresponding to dashed boxes in the figures). Those skilled in the art can make appropriate variations according to the above content, rather than being limited by the disclosure of the foregoing accompanying drawings.

The above embodiments merely provide illustrative descriptions of the embodiments of the present application. However, the present application is not limited thereto, and appropriate variations may be made on the basis of the above embodiments. For example, each of the above embodiments may be used independently, or one or more among the above embodiments may be combined.

According to the above embodiment, the vital sign state information of the subject is determined based on the duration during which the value of the vital sign-related parameter of the subject is within the preset value range and the coefficient corresponding to the preset value range, and a degree of abnormality of the vital sign-related parameter of the subject during the preset time period can be quantitatively reflected by means of the vital sign state information, so that more detailed and accurate information can be provided for a user.

Further provided in the embodiments of the present application is an information processing apparatus; content the same as that in the previous embodiments is not described again here. FIG. 4 is a schematic diagram of an information processing apparatus according to an embodiment of the present application. As shown in FIG. 4, the information processing apparatus 400 includes: a processor 410. The processor 410 is configured to execute the foregoing information processing method. For example, the processor 410 may be configured to perform the following controls: acquiring a value of a vital sign-related parameter of a subject during a preset time period; determining a duration during which the value of the vital sign-related parameter is within a preset value range; and, based on the duration corresponding to the preset value range and a coefficient corresponding to the preset value range, determining vital sign state information of the subject.

For the information processing method, reference may be made to the foregoing embodiments, the contents of which are incorporated herein and will not be described again.

As shown in FIG. 4, the information processing apparatus 400 may further include a memory 420. The memory 420 may store data and a program and be coupled to the processor 410. For example, the processor 410 may be configured to execute the program in the memory 420 to implement the foregoing signal processing method. It should be noted that this figure is exemplary; and another type of structure may be used to supplement or replace this structure to implement a related function.

As shown in FIG. 4, the information processing apparatus 400 may further include: a communication module 430, an input unit 440, a display 450, and a power source 460. The functions of the foregoing components are similar to those in the prior art. Details are not described herein again. It should be noted that the information processing apparatus 400 does not necessarily have to include all of the components shown in FIG. 4, and the foregoing components are not essential. In addition, the information processing apparatus 400 may further include components not shown in FIG. 4, for which reference may be made to the prior art.

In addition, for simplicity, the above figures only exemplarily illustrate connection relationships or signal directions between various components or modules, but it should be clear to those skilled in the art that various related technologies such as bus connection may be used. The various components or modules may be implemented by means of hardware facilities such as a processor, a memory, a transmitter and a receiver. The implementation of the present application is not limited thereto.

According to the above embodiment, the vital sign state information of the subject is determined based on the duration during which the value of the vital sign-related parameter of the subject is within the preset value range and the coefficient corresponding to the preset value range, and a degree of abnormality of the vital sign-related parameter of the subject during the preset time period can be quantitatively reflected by means of the vital sign state information, so that more detailed and accurate information can be provided for a user.

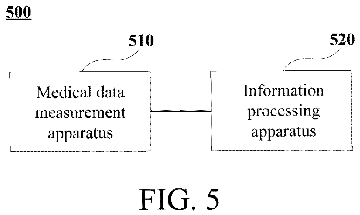

Further provided in the embodiments of the present application is an information processing system, of which the same contents as those in the previous embodiments are not described again here. FIG. 5 is a schematic diagram of an information processing system according to an embodiment of the present application. As shown in FIG. 5, the information processing system 500 includes: a medical data measurement apparatus 510 and an information processing apparatus 520. The medical data measurement apparatus 510 measures a value of a vital sign-related parameter of a subject; and the information processing apparatus 520 acquires the value of the vital sign-related parameter of the subject during a preset time period, determines a duration during which the value of the vital sign-related parameter is within a preset value range, and determines vital sign state information of the subject based on the duration corresponding to the preset value range and a coefficient corresponding to the preset value range.

The information processing apparatus 520 may be the information processing apparatus 400 described in the previous embodiments, the contents of which are incorporated herein and will not be further described.

In some embodiments, the medical data measurement apparatus 510 may be a variety of apparatuses capable of acquiring the value of the vital sign-related parameter of the subject; this is not specifically limited in the present application.

In addition, for simplicity, the above figures only exemplarily illustrate connection relationships or signal directions between various components or modules, but it should be clear to those skilled in the art that various related technologies such as bus connection may be used. The various components or modules may be implemented by means of hardware facilities such as a processor, a memory, a transmitter and a receiver. The implementation of the present application is not limited thereto.

According to the above embodiment, the vital sign state information of the subject is determined based on the duration during which the value of the vital sign-related parameter of the subject is within the preset value range and the coefficient corresponding to the preset value range, and a degree of abnormality of the vital sign-related parameter of the subject during the preset time period can be quantitatively reflected by means of the vital sign state information, so that more detailed and accurate information can be provided for a user.

Further provided in the embodiments of the present application is a computer-readable program, wherein the program, when executed, causes a computer to execute, in the information processing apparatus, the information processing method described in the foregoing embodiments.

Further provided in the embodiments of the present application is a storage medium storing a computer-readable program, wherein the computer-readable program causes a computer to execute, in the information processing apparatus, the information processing method described in the foregoing embodiments.

The above apparatus and method of the present application can be implemented by hardware, or can be implemented by hardware in combination with software. The present application relates to such a computer-readable program that when executed by a logic component, the program causes the logic component to implement the foregoing apparatus or a constituent component, or causes the logic component to implement various methods or steps as described above. The present application further relates to a storage medium for storing the above program, such as a hard disk, a disk, an optical disk, a DVD, a flash memory, etc.

The method/apparatus described in view of the embodiments of the present application may be directly embodied as hardware, a software module executed by a processor, or a combination of the two. For example, one or more of the functional block diagrams and/or one or more combinations of the functional block diagrams shown in the drawings may correspond to either respective software modules or respective hardware modules of a computer program flow. The foregoing software modules may respectively correspond to the steps shown in the figures. The foregoing hardware modules can be implemented, for example, by firming the software modules using a field-programmable gate array (FPGA).

The software modules may be located in a RAM, a flash memory, a ROM, an EPROM, an EEPROM, a register, a hard disk, a portable magnetic disk, a CD-ROM, or any other form of storage medium known in the art. The storage medium may be coupled to a processor, so that the processor can read information from the storage medium and can write information into the storage medium. Alternatively, the storage medium may be a constituent component of the processor. The processor and the storage medium may be located in an ASIC. The software module may be stored in a memory of a mobile terminal, and may also be stored in a memory card that can be inserted into a mobile terminal. For example, if a device (such as a mobile terminal) uses a large-capacity MEGA-SIM card or a large-capacity flash memory apparatus, the software modules can be stored in the MEGA-SIM card or the large-capacity flash memory apparatus.

One or more of the functional blocks and/or one or more combinations of the functional blocks shown in the accompanying drawings may be implemented as a general-purpose processor, a digital signal processor (DSP), an application-specific integrated circuit (ASIC), a field-programmable gate array (FPGA) or other programmable logic devices, discrete gate or transistor logic devices, a discrete hardware assembly, or any appropriate combination thereof for implementing the functions described in the present application. The one or more functional blocks and/or the one or more combinations of the functional blocks shown in the accompanying drawings may also be implemented as a combination of computing devices, such as a combination of a DSP and a microprocessor, multiple microprocessors, one or more microprocessors in communication combination with a DSP, or any other such configuration.

The present application is described above with reference to specific embodiments. However, it should be clear to those skilled in the art that the foregoing description is merely illustrative and is not intended to limit the scope of protection of the present application. Various variations and modifications may be made by those skilled in the art according to the spirit and principle of the present application, and these variations and modifications also fall within the scope of the present application.

The above embodiments merely provide illustrative descriptions of the embodiments of the present application. However, the present application is not limited thereto, and appropriate variations may be made on the basis of the above embodiments. For example, each of the above embodiments may be used independently, or one or more among the above embodiments may be combined.

Preferred embodiments of the present application are described above with reference to the accompanying drawings. Many features and advantages of the implementations are clear according to the detailed description, and therefore the appended claims are intended to cover all these features and advantages that fall within the true spirit and scope of these implementations. In addition, as many modifications and changes could be easily conceived of by those skilled in the art, the embodiments of the present application are not limited to the illustrated and described precise structures and operations, but can encompass all appropriate modifications, changes, and equivalents that fall within the scope of the implementations.

## Claims

1. An information processing method, executed by a computer, **characterized in that** the method comprises:
acquiring a value of a vital sign-related parameter of a subject during a preset time period;
determining a duration during which the value of the vital sign-related parameter is within a preset value range; and
based on the duration corresponding to the preset value range and a coefficient corresponding to the preset value range, determining vital sign state information of the subject.

2. The method according to claim 1, wherein
the duration comprises: a cumulative duration within the preset time period during which the value of the vital sign-related parameter is within the preset value range; or, a duration within the preset time period during which the value of the vital sign-related parameter remains continuously within the preset value range in a time dimension.

3. The method according to claim 1, wherein
the vital sign state information comprises: a result obtained by weighting the duration corresponding to the preset value range using the coefficient corresponding to the preset value range.

4. The method according to claim 3, wherein
when there are a plurality of preset value ranges, the vital sign state information comprises a value obtained by performing weighted summation or weighted averaging on the duration corresponding to the preset value range, using the coefficient corresponding to the preset value range.

5. The method according to claim 1, wherein
the preset value range is a range other than a target value range, wherein the vital sign-related parameter is in a normal state when the value of the vital sign-related parameter is within the target value range, and the vital sign-related parameter is in an abnormal state when the vital sign-related parameter is within the preset value range.

6. The method according to claim 5, wherein
the coefficient corresponding to the preset value range is related to a distance between the preset value range and the target value range.

7. The method according to claim 6, wherein
a greater distance between the preset value range and the target value range indicates a larger coefficient corresponding to the preset value range; or
a smaller distance between the preset value range and the target value range indicates a smaller coefficient corresponding to the preset value range.

8. The method according to claim 6, wherein
the coefficient corresponding to the preset value range is further related to a change trend of the value of the vital sign-related parameter relative to the target value range during a time period during which the value of the vital sign-related parameter is within the preset value range.

9. The method according to claim 8, wherein
the change trend comprises a first trend and a second trend, wherein the first trend comprises a trend that the value of the vital sign-related parameter approaches the target value range, the second trend comprises a trend that the value of the vital sign-related parameter deviates from the target value range, and a coefficient corresponding to the first trend is less than or equal to a coefficient corresponding to the second trend.

10. The method according to claim 6, wherein
the coefficient corresponding to the preset value range is further related to a distance between a time when the value of the vital sign-related parameter is within the preset value range and a current time.

11. The method according to claim 10, wherein
the value of the vital sign-related parameter is within the preset value range during a first time period and a second time period spaced from the first time period, the first time period being a time period earlier than the second time period, and a coefficient corresponding to the first time period being less than or equal to a coefficient corresponding to the second time period.

12. The method according to claim 6, wherein
the coefficient corresponding to the preset value range is further related to a degree of dispersion of the value of the vital sign-related parameter during the preset time period.

13. The method according to claim 12, wherein
a greater degree of dispersion of the value of the vital sign-related parameter during the preset time period indicates a larger coefficient corresponding to the preset value range; and
a lesser degree of dispersion of the value of the vital sign-related parameter during the preset time period indicates a smaller coefficient corresponding to the preset value range.

14. The method according to claim 1, wherein
the vital sign-related parameter comprises one or more parameters.

15. The method according to claim 14, further comprising:
when the vital sign-related parameter comprises a plurality of parameters, separately determining vital sign state information corresponding to each parameter.
